# FASCICULE DE BREVET EUROPEEN

(11) **EP 0 815 834 B1**
(45) Date de publication et mention de la délivrance du brevet: **20.12.2000**
(21) Numéro de dépôt: 97107303.6
(22) Date de dépôt: 13.11.1995
(51) Int. Cl.: A61K 7/42

(54) **Compositions cosmétiques filtrantes photostabilisées comprenant un dérivé de dibenzoylméthane et un composé amidé**
Photostabile filtrierende kosmetische Zusammensetzungen enthaltend ein Dibenzoylmethanderivat und eine Amid-Verbindung
Photostable cosmetic sunscreen compositions containing a dibenzoylmethan derivative and an amide compound

(30) Priorité: 12.12.1994 FR 9414930
(43) Date de publication de la demande: 07.01.1998
(62) Demande divisionnaire de: 95402536.7
(73) Titulaire: L'OREAL, 75008 Paris (FR)
(72) Inventeur: Ascione, Jean-Marc, Hoboken, New Jersey 07030 (US); Forestier, Serge, 77410 Claye Souilly (FR); Sterle, Pascal, 95230 Soisy S/Montmorency (FR)
(74) Mandataire: Andral, Christophe André Louis

(56) Documents cités:
- EP-A- 0 396 422
- EP-A- 0 521 651
- EP-A- 0 604 249
- WO-A-94/04131
- WO-A-94/14410
- FR-A- 2 440 933
- US-A- 4 810 489
- US-A- 4 866 159

## Description

La présente invention concerne de nouvelles compositions cosmétiques à usage topique spécifiquement destinées à la photoprotection de la peau et/ou des cheveux contre le rayonnement ultraviolet (compositions ci-après dénommées plus simplement compositions antisolaires ou compositions filtrantes) ainsi que leur utilisation dans l'application cosmétique susmentionnée. Plus précisément encore, elle concerne des compositions antisolaires photostables à l'égard des UV qui comprennent, dans un support cosmétiquement acceptable, au moins un composé choisi parmi les dérivés du dibenzoylméthane à titre de filtre solaire organique actif dans l'UV-A, associé à au moins un composé amidé à titre d'agent photostabilisant.

On sait que les rayons UV-A, de longueurs d'onde comprises entre 320 et 400 nm, provoquent le brunissement de la peau, mais qu'ils sont également susceptibles d'induire à la longue une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent en outre le déclenchement de la réaction érythémateuse ou amplifient cette réaction chez certains sujets et peuvent même être à l'origine de réactions photo-toxiques ou photo-allergiques. Il est donc souhaitable de filtrer le rayonnement UV-A.

De nombreux filtres organiques solaires capables d'absorber plus ou moins sélectivement les rayons UV-A nocifs ont été proposés à ce jour dans le domaine de la cosmétique.

A cet égard, une famille de filtres UV-A particulièrement intéressante est actuellement constituée par les dérivés du dibenzoylméthane, et notamment le 4-(ter.-butyl) 4'-méthoxy dibenzoylméthane, qui présentent en effet un fort pouvoir d'adsorption intrinsèque. Ces dérivés du dibenzoylméthane, qui sont maintenant des produits bien connus en soi à titre de filtres actifs dans l'UV-A, sont notamment décrits dans les demandes de brevets français FR-A- 2 326 405 et FR-A- 2 440 933, ainsi que dans la demande de brevet européen EP-A- 0 114 607 ; le 4-(ter.-butyl) 4'-méthoxy dibenzoylméthane est par ailleurs actuellement proposé à la vente sous la dénomination commerciale de "PARSOL 1789" par la Société GIVAUDAN.

Malheureusement, il se trouve que les dérivés du dibenzoylméthane sont des produits relativement sensibles au rayonnement ultraviolet (surtout UV-A), c'est à dire, plus précisément, qu'ils présentent une facheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

La photostabilisation des dérivés du dibenzoylméthane vis-à-vis du rayonnement UV constitue, à ce jour, un problème qui n'a pas encore été résolu de manière complètement satisfaisante.

On connaît dans les demandes de brevet WO94/04131 et EP-A-521 651 des formulations cosmétiques filtrantes à base de dérivés de dibenzoylméthane et de composés amidés tensio-actifs tels que les amides d'acides gras; le lactamide monoéthanolamine, l'acétamide monoéthanolamine, les mono ou dialkylalcanolamides (ie : coco- mono ou diéthanolamide, coco mono isopropanolamide) ; les alkyl amidopropylbétaines (cocomidopropylbétaine). On connaît dans les demandes de brevet EP-A-604 249 et WO94/04131 des formulations cosmétiques fil-. trantes à base de dérivés de dibenzoylméthane et de polymères épaississants amidés ioniques comme le polymère réticulé acrylamide/acide 2-acrylamido 2-méthylpropane sulfonique en émulsion huile/eau avec de l'isoparaffine et le laureth-7 (SEPIGEL) ou les copolymères acrylamide/acrylate cationique (SALCARE SC 92). On connaît dans les demandes de brevet FR 2 440 933, EP396 422 et WO94/04131 des formulations cosmétiques filtrantes à base de dérivés de dibenzoylméthane et de composés amidés actifs hydrosolubles tels que l'urée, la niacinamide ou l'iodopropynyl-carbamate. On connaît dans les demandes de brevet WO94/14410 des formulations cosmétiques filtrantes à base de polymères filtres anioniques comprenant au moins un monomère acrylique dibenzoylméthane filtrant les UV-A et au moins un monomère acrylique benzoylamide filtrant les UV-B. On connaît également dans le brevet US 4,866, 159 des compositions solaires comprenant des polyaminoamides filtrants les radiations UV-B pouvant contenir en autres également des dérivés de dibenzoylméthane.

Or, la Demanderesse vient maintenant de découvrir, de façon inattendue et surprenante, qu'en associant aux dérivés du dibenzoylméthane mentionnés ci-dessus une quantité efficace d'au moins un composé amidé, non-ionique, non-hydrosoluble et ne présentant pas de propriétés émulsionnantes, il est possible d'améliorer de manière substantielle et remarquable, la stabilité photochimique (ou photostabilité) de ces mêmes dérivés du dibenzoylméthane. Cette découverte, essentielle, est à la base de la présente invention.

Une autre difficulté, indépendante de celle évoquée ci-avant, rencontrée avec les dérivés de dibenzoylméthane est qu'il s'agit de filtres lipophiles présentant la particularité mais aussi le désavantage d'être solides à température ambiante. De ce fait, leur utilisation dans une composition cosmétique antisolaire implique certaines contraintes au niveau de leur formulation et de leur mise en oeuvre, en particulier lorsqu'il s'agit de trouver des solvants permettant de les solubiliser correctement, seuls ou conjointement avec d'autres filtres.

A cet égard, on fait aujourd'hui le plus souvent appel à des huiles comme des esters et plus particulièrement des benzoates d'alkyles en C₁₂-C₁₅ ("FINSOLV TN" de chez Finetex), ou à des triglycérides et notamment des triglycérides d'acides gras en C₈-C₁₂ ("MIGLYOL 812" de chez Hüls), mais ces différents produits possèdent des propriétés solubilisantes vis à vis des filtres susmentionnés qui peuvent apparaitre comme encore insuffisantes.

Or, il a été justement trouvé, et il s'agit là de l'un des avantages supplémentaires attachés à la présente invention, que certains des composés amidés utilisables dans le cadre de la présente invention à titre d'agents photostabilisants constituent par ailleurs, de façon également très surprenante, des solvants particulièrement remarquables pour les filtres du type dérivés du dibenzoylméthane comme par exemple le 4-(ter.-butyl) 4'-méthoxy dibenzoylméthane, ces dérivés présentant en effet dans ces composés amidés des solubilités extrêmement élevées, et en tous cas nettement supérieures à celles obtenues avec tous les autres solvants usuels utilisés à ce jour, ce qui permet, à quantité égale de solvant, de mettre en oeuvre des quantités plus importantes de filtres.

Conformément à un objet, il est proposé de nouvelles compositions cosmétiques filtrantes photostables destinées à la protection de la peau et/ou des cheveux contre le rayonnement ultraviolet solaire, en particulier le rayonnement, telles que définies dans la revendication 1.

D'autres caractéristiques, aspects et avantages de la présente invention apparaitront à la lecture de la description détaillée qui va suivre.

Comme indiqué précédemment, les dérivés du dibenzoylméthane destinés à être photostabilisés dans le cadre de la présente invention sont des produits déjà bien connus en soi et décrits notamment dans les documents FR-A- 2 326 405, FR-A-2 440 933 et EP-A- 0 114 607 précités, documents dont les enseignements sont, pour ce qui touche à la définition même de ces produits, totalement inclus à titre de références dans la présente description.

Selon la présente invention, on peut bien entendu mettre en oeuvre un ou plusieurs dérivés du dibenzoylméthane.

Parmi les dérivés du dibenzoylméthane rentrant particulièrement bien dans le cadre de la présente invention, on peut notamment citer, de manière non limitative :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane

Parmi les dérivés du dibenzoylméthane mentionnés ci-dessus, on préfère tout particulièrement, selon la présente invention, mettre en oeuvre le 4-(ter.-butyl) 4'-méthoxy dibenzoylméthane, notamment celui proposé à la vente sous la dénomination commerciale de "PARSOL 1789" par la Société GIVAUDAN, ce filtre répondant donc à la formule développée suivante :

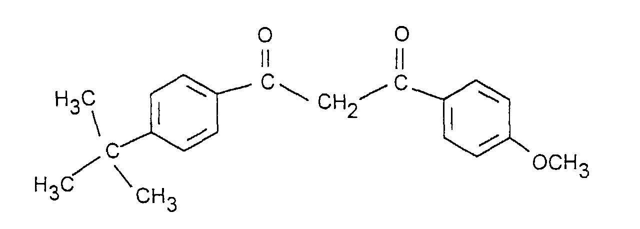

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

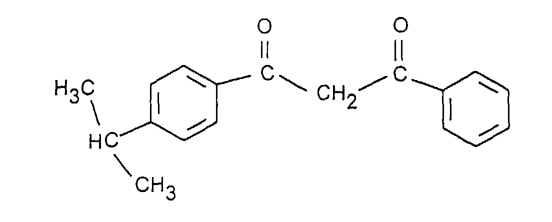

Le ou les dérivés du dibenzoylméthane peuvent être présents dans les compositions conformes à l'invention, ou dans les compositions destinées à être stabilisées conformément au procédé de l'invention, à des teneurs qui sont généralement comprises entre 0,01 % et 10 % en poids, et de de préférence à des teneurs comprises entre 0,1 % et 6 % en poids, par rapport au poids total de la composition.

Au sens de la présente invention, on entend par composé amidé tout composé présentant dans sa structure chimique au moins un groupement (ou fonction) amide

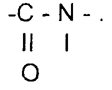

Par quantité efficace de composé amidé, on entend une quantité suffisante pour obtenir une amélioration notable et significative de la photostabilité du ou des dérivés du dibenzoylméthane contenus dans la composition. Cette quantité minimale en agent stabilisant à mettre en oeuvre, qui peut varier selon la nature du support cosmétiquement acceptable retenu pour la composition, peut être déterminée sans aucune difficulté au moyen d'un test classique de mesure de photostabilité, tel que celui donné dans les exemples ci-après.

D'une manière générale, le ou les composés amidés peuvent ainsi être présents dans les compositions conformes à l'invention, ou utilisés dans le procédé conforme à l'invention, à des teneurs qui sont généralement comprises entre 0,01 % et 50 % en poids, et de de préférence à des teneurs comprises entre 0,1 % et 30 % en poids, par rapport au poids total de la composition.

Les composés amidés visés par la présente invention sont ceux répondant à la formule (1) suivante : 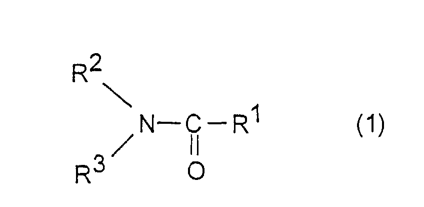 dans laquelle les radicaux R¹, R² et R³, qui peuvent être identiques ou différents, représentent l'hydrogène ou des radicaux hydrocarbonés monovalents, saturés ou insaturés, aliphatiques, cycloaliphatiques ou cycliques, éventuellement fonctionnalisés, contenant de 1 à 30 atomes de carbone, de préférence de 1 à 22 atomes de carbone, bornes incluses, étant entendu que, dans cette formule, le radical R¹ peut former avec le radical R² ou avec le radical R³ un cycle contenant inclusivement de 5 à 18 atomes de carbone, et que les radicaux R² et R³ peuvent ensemble former un cycle contenant de 5 à 18 atomes de carbone, bornes incluses.

Comme exemples de radicaux hydrocarbonés saturés aliphatiques, on peut notamment citer les radicaux alkyles, linéaires ou ramifiés, substitués ou non, en C₁-C₃₀, de préférence en C₁-C₂₂, et en particulier les radicaux méthyle, éthyle, n-propyle, isopropyle, n-butyle, isobutyle, ter.-butyle, pentyle, n-amyle, isoamyle, néopentyle, n-hexyle, n-heptyle, n-octyle, 2-éthylhexyle, ter.-octyle, décyle, lauryle et octadécyle.

A titre d'exemples de radicaux hydrocarbonés saturés cycliques, on peut notamment citer les radicaux cyclopentyle et cyclohexyle, éventuellement substitués, en particulier par des radicaux alkyles.

Comme exemples de radicaux hydrocarbonés insaturés aliphatiques, on peut notamment citer les radicaux alcényles ou alcynyles, linéaires ou ramifiés, substitués ou non, en C₂-C₃₀, de préférence en C₂-C₂₂, et particulier les radicaux vinyle, allyle, oléyle et linoléyle.

Comme exemples de radicaux hydrocarbonés insaturés cycliques, on peut notamment citer les radicaux aryles tels que phényle et naphtyle, éventuellement substitués, en particulier par des alkyles, comme par exemple le radical tolyle, et à titre d'exemples de radicaux cycloaliphatiques insaturés, on peut citer plus particulièrement les radicaux benzyle et phényléthyle.

Par radicaux fonctionnalisés, on entend plus particulièrement des radicaux comportant dans leur structure chimique, tant dans la chaine principale que sur un chainon secondaire, un ou plusieurs groupements fonctionnels du type notamment esters, éthers, alcools, amines, mais de préférence esters.

Parmi les composés amidés de formule (I) convenant bien à la présente invention, on préfère plus particulièrement mettre en oeuvre les composés présentant au moins l'une, et encore plus préférentiellement l'ensemble, des caractéristiques suivantes :
- le composé amidé est un amide N-substitué,
- R¹ est un radical alkyle, linéaire ou ramifié, de préférence en C₁-C₂₂ et encore plus préférentiellement en C₁-C₁₂, ou bien encore un radical phényle lui-même éventuellement substitué par un ou plusieurs radicaux alkyles, linéaires ou ramifiés, en C₁-C₁₂,
- R² est un radical alkyle, linéaire ou ramifié, de préférence en C₁-C₂₂ et encore plus préférentiellement en C₁-C₁₂,
- R³ est un radical alkyle, linéaire ou ramifié, choisi au sein de ceux définis pour R², ou bien encore représente un radical monovalent à fonction ester répondant à la formule (2) suivante : 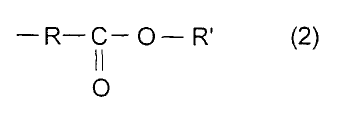 dans laquelle R et R', qui peuvent être identiques ou différents, représentent deux radicaux hydrocarbonés, de préférence de type alkyle, contenant de 1 à 12 atomes de carbone, de préférence de 1 à 8 atomes de carbone.

Selon un mode particulier de réalisation de la présente invention, les composés amidés, en particulier ceux précédemment définis, que l'on met en oeuvre sont ceux dans lesquels le ou les dérivés du dibenzoylméthane à stabiliser présentent une bonne solubilité.

Selon un autre mode particulier de réalisation de la présente invention, les composés amidés, en particulier ceux précédemment définis, que l'on met en oeuvre sont ceux qui présentent une bonne solubilité dans les phases grasses habituellement utilisées pour la préparation de supports cosmétiquement acceptables.

Selon un autre mode particulier de réalisation de la présente invention, les composés amidés, en particulier ceux précédemment définis, sont mis en oeuvre en une quantité suffisante pour solubiliser à eux seuls le ou les dérivés du dibenzoylméthane à stabiliser.

Selon un autre mode particulier de réalisation de la présente invention, les composés amidés, en particulier ceux précédemment définis, que l'on met en oeuvre sont ceux qui sont dépourvus, ou substantiellement dépourvus, de toute propriété émulsionnante.

Les compositions cosmétiques antisolaires photostables selon l'invention peuvent bien entendu contenir, outre les dérivés du dibenzoylméthane, un ou plusieurs filtres solaires complémentaires actifs dans l'UVA et/ou l'UVB, hydrophiles ou lipophiles. La présence de filtres complémentaires actifs dans l'UV-B (longueurs d'ondes comprise entre 280nm et 320 nm environ) permet ainsi de disposer de compositions finales capables de filtrer l'ensemble des rayons UV.

Les compositions de l'invention peuvent aussi comprendre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les pigments (minéraux ou organiques), les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions. Bien entendu, tous les ingrédients supplémentaires susceptibles d'être introduits dans les compositions conformes à l'invention doivent être tels qu'ils ne perturbent ou n'altèrent substantiellement pas l'effet de photostabilisation exercé par les composés amidés sur les dérivés du dibenzoylméthane.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose, l'hydroxypropylméthyl cellulose et l'hydroxyéthyl cellulose.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).
Les compositions cosmétiques photostables de l'invention peuvent être utilisées comme compositions protectrices de l'épiderme humain ou des cheveux contre les rayons ultraviolets, comme compositions antisolaires ou encore comme produits de maquillage.

Lorsque les compositions cosmétiques selon l'invention sont utilisées pour la protection de l'épiderme humain contre les rayons UV, ou comme composition antisolaire, elles peuvent se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de bâtonnet solide, de stick, de mousse aérosol ou de spray.

Lorsque les compositions cosmétiques selon l'invention sont utilisées pour la protection des cheveux, elles peuvent se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque les compositions sont utilisées comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elles peuvent se présenter sous forme solide ou pâteuse, anhydre ou aqueuse, comme des émulsions huile dans eau ou eau dans huile, des dispersions vésiculaires non ioniques ou encore des suspensions.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique photostable telle que définie ci-dessus.

## Revendications

1. Composition cosmétique filtrante photostable pour la photoprotection par voie topique de la peau et/ou des cheveux contre le rayonnement ultraviolet, en particulier le rayonnement solaire, du type comprenant, dans un support cosmétiquement acceptable, au moins un dérivé du dibenzoylméthane, caractérisée par le fait qu'elle comprend en outre une quantité efficace d'au moins un composé amidé non-ionique, non-hydrosoluble et ne présentant pas de propriétés émulsionnantes choisi au sein de ceux répondant à la formule (1) suivante : 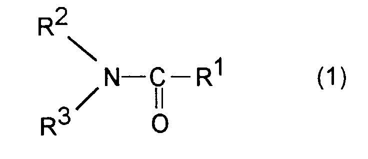 dans laquelle R¹, R² et R³, qui peuvent être identiques ou différents, représentent l'hydrogène ou des radicaux hydrocarbonés monovalentes, saturés ou insaturés, aliphatiques ou cycloaliphatiques ou cycliques, si fonctionnalisés, les groupes fonctionnels étant choisis parmi esters, éthers, alcools, amines contenant inclusivement de 1 à 30 atomes de carbone, étant entendu que R¹ peut former soit avec R² soit avec R³ un cycle contenant inclusivement de 5 à 18 atomes de carbone, et que R² et R³ peuvent former ensemble un cycle contenant inclusivement de 5 à 18 atomes de carbone ; à l'exception des composés amides N,N-disubstitués.

2. Composition selon la revendication 1, caractérisée par le fait que ledit dérivé du dibenzoylméthane est choisi parmi
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane

3. Composition selon la revendication 2, caractérisée par le fait que ledit dérivé du dibenzoylméthane est est le 4-(ter.-butyl) 4'-méthoxy dibenzoylméthane ou le 4-isopropyl-dibenzoylméthane.

4. Composition selon la revendication 3, caractérisée par le fait que ledit dérivé du dibenzoylméthane est le 4-(ter.-butyl) 4'-méthoxy dibenzoylméthane.

5. Composition selon l'une quelconque des revendication 1 à 4, caractérisée par le fait que le composé amidé est N-substitué.

6. Composition selon l'une quelconque des revendications 1 à 5, caractérisée par le fait que lesdits radicaux hydrocarbonés monovalents contiennent de 1 à 22 atomes de carbone, inclusivement.

7. Composition selon l'une quelconque des revendications 1 à 6, caractérisée par le fait que le radical R¹ est un radical alkyle, linéaire ou ramifié, de préférence en C₁-C₁₂, ou un radical phényle éventuellement substitué par un ou plusieurs radicaux alkyles, linéaires ou ramifiés, en C₁-C₁₂.

8. Composition selon l'une quelconque des revendication 1 à 7, caractérisée par le fait que le radical R² est un radical alkyle, linéaire ou ramifié, de préférence en C₁-C₁₂.

9. Composition selon l'une quelconque des revendications 1 à 7, caractérisée par le fait R³ est un radical alkyle, linéaire ou ramifié, choisi au sein de ceux définis pour R², ou bien encore représente un radical monovalent à fonction ester répondant à la formule (2) suivante : 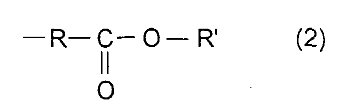 dans laquelle R et R', identiques ou différents, représentent un radical hydrocarboné contenant de 1 à 12 atomes de carbone.

10. Composition selon la revendication 9, caractérisé par le fait que ledit radical hydrocarboné contient de 1 à 8 atomes de carbone.

11. Composition selon l'une des revendications 9 ou 10, caractérisé par le fait que ledit radical hydrocarboné est de type alkyle.

12. Composition selon l'une quelconque des revendications 1 à 11, caractérisée par le fait que la teneur en dérivé(s) du dibenzoylméthane est comprise entre entre 0,01 % et 10 % en poids par rapport au poids total de la composition.

13. Composition selon la revendication 12, caractérisées par le fait que ladite teneur est comprise entre 0,1 % et 6 % en poids par rapport au poids total de la composition.

14. Composition selon l'une quelconque des revendications 1 à 13, caractérisée par le fait que la teneur en composé(s) amidé(s) est comprise entre 0,01 % et 50 % en poids par rapport au poids total de la composition.

15. Composition selon la revendication 14, caractérisée par le fait que ladite teneur est comprise entre 0,1 % et 30 % en poids par rapport au poids total de la composition.

## Patentansprüche

1. Photostabile, kosmetische Filterzusammensetzung zum Lichtschutz der Haut und/oder der Haare gegen UV-Strahlung und insbesondere gegen Sonnenlicht auf topischem Wege, die in einem kosmetisch akzeptablen Träger mindestens ein Dibenzoylmethanderivat enthält, dadurch gekennzeichnet, daß sie ferner mindestens eine nichtionische, nicht wasserlösliche, amidierte Verbindung enthält, die keine emulgierenden Eigenschaften aufweist und die unter den Verbindungen der folgenden Formel (1) ausgewählt ist: 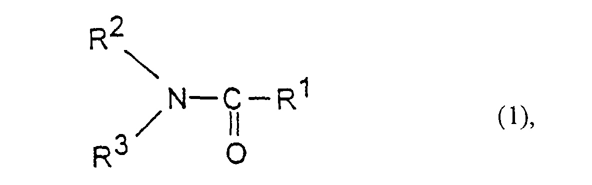 worin die Gruppen R¹, R² und R³, die identisch oder voneinander verschieden sein können, Wasserstoff oder einwertige, gesättigte oder ungesättigte, aliphatische, cycloaliphatische oder cyclische, funktionalisierte Kohlenwasserstoffgruppen, die 1 bis 30 Kohlenstoffatome aufweisen, bedeuten, wobei die funktionellen Gruppen unter den Estern, Ethern, Alkoholen und Aminen ausgewählt sind, mit der Maßgabe, daß R¹ entweder mit R² oder mit R³ einen Ring bilden kann, der 5 bis 18 Kohlenstoffatome aufweist, und R² und R³ gemeinsam einen Ring bilden können, der 5 bis 18 Kohlenstoffatome aufweist; mit Ausnahme von N,N-disubstituierten amidierten Verbindungen.

2. Zusammensetzung nach Anspruch 1, dadurch gekennzeichnet, daß das Dibenzoylmethanderivat unter
- 2-Methyl-dibenzoylmethan,
- 4-Methyl-dibenzoylmethen,
- 4-Isopropyl-dibenzoylmethan,
- 4-*tert*.-Butyl-dibenzoylmethan,
- 2,4-Dimethyl-dibenzoylmethan,
- 2,5-Dimethyl-dibenzoylmethan,
- 4,4'-Diisopropyl-dibenzoylmethan,
- 4-*tert*.-Butyl-4'-methoxy-dibenzoylmethan,
- 2-Methyl-5-isopropyl-4'-methoxy-dibenzoylmethan,
- 2-Methyl-5-*tert.*-butyl-4'-methoxy-dibenzoylmethan,
- 2,4-Dimethyl-4'-methoxy-dibenzoylmethan, und
- 2,6-Dimethyl-4-*tert.*-butyl-4'-methoxy-dibenzoylmethan
ausgewählt ist.

3. Zusammensetzung nach Anspruch 2, dadurch gekennzeichnet, daß es sich bei dem Dibenzoylmethanderivat um 4-*tert*.-Butyl-4'-methoxy-dibenzoylmethan oder 4-Isopropyl-dibenzoylmethan handelt.

4. Zusammensetzung nach Anspruch 3, dadurch gekennzeichnet, daß es sich bei dem Dibenzoylmethanderivat um 4-tert.-Butyl-4'-methoxy-dibenzoylmethan handelt.

5. Zusammensetzung nach einem der Ansprüche 1 bis 4, dadurch gekennzeichnet, daß die amidierte Verbindung N-substituiert ist.

6. Zusammensetzung nach einem der Ansprüche 1 bis 5, dadurch gekennzeichnet, daß die einwertigen Kohlenwasserstoffgruppen 1 bis 22 Kohlenstoffatome aufweisen.

7. Zusammensetzung nach einem der Ansprüche 1 bis 6, dadurch gekennzeichnet, daß die Gruppe R¹ eine geradkettige oder verzweigte Alkylgruppe, die vorzugsweise 1 bis 12 Kohlenstoffatome aufweist, oder eine Phenylgruppe ist, die gegebenenfalls mit einer oder mehreren, geradkettigen oder verzweigten C₁₋₁₂-Alkylgruppen substituiert ist.

8. Zusammensetzung nach einem der Ansprüche 1 bis 7, dadurch gekennzeichnet, daß die Gruppe R² eine geradkettige oder verzweigte Alkylgruppe ist, die vorzugsweise 1 bis 12 Kohlenstoffatome aufweist.

9. Zusammensetzung nach einem der Ansprüche 1 bis 7, dadurch gekennzeichnet, daß die Gruppe R³ eine geradkettige oder verzweigte Alkylgruppe, die unter den bezüglich R² definierten Gruppen ausgewählt ist, oder eine einwertige Gruppe mit Estergruppe ist, die der folgenden Formel (2) entspricht: 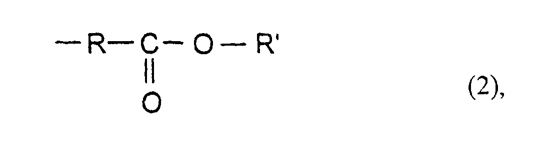 worin die Gruppen R und R', die identisch oder voneinander verschieden sind, eine Kohlenwasserstoffgruppe mit 1 bis 12 Kohlenstoffatomen bedeuten.

10. Zusammensetzung nach Anspruch 9, dadurch gekennzeichnet, daß die Kohlenwasserstoffgruppe 1 bis 8 Kohlenstoffatome aufweist.

11. Zusammensetzung nach einem der Ansprüche 9 oder 10, dadurch gekennzeichnet, daß die Kohlenwasserstoffgruppe eine Gruppe vom Alkyltyp ist.

12. Zusammensetzung nach einem der Ansprüche 1 bis 11, dadurch gekennzeichnet, daß der Mengenanteil des Dibenzoylmethanderivats oder der Dibenzoylmethanderivate im Bereich von 0,01 bis 10 Gew.-%, bezogen auf das Gesamtgewicht der Zusammensetzung, liegt.

13. Zusammensetzung nach Anspruch 12, dadurch gekennzeichnet, daß der Mengenanteil im Bereich von 0,1 bis 6 Gew.-%, bezogen auf das Gesamtgewicht der Zusammensetzung, liegt.

14. Zusammensetzung nach einem der Ansprüche 1 bis 13, dadurch gekennzeichnet, daß der Mengenanteil der amidierten Verbindung(en) im Bereich von 0,01 bis 50 Gew.-%, bezogen auf das Gesamtgewicht der Zusammensetzung, liegt.

15. Zusammensetzung nach Anspruch 14, dadurch gekennzeichnet, daß der Mengenanteil im Bereich von 0,1 bis 30 Gew.-%, bezogen auf das Gesamtgewicht der Zusammensetzung, liegt.

## Claims

1. Photostable screening cosmetic composition for the topical photoprotection of the skin and/or hair against ultraviolet radiation, in particular solar radiation, of the type comprising, in a cosmetically acceptable vehicle, at least one dibenzoylmethane derivative, characterized in that it additionally comprises an effective amount of at least one nonionic and water-insoluble amide compound which does not exhibit emulsifying properties chosen from those corresponding to the following formula (1): 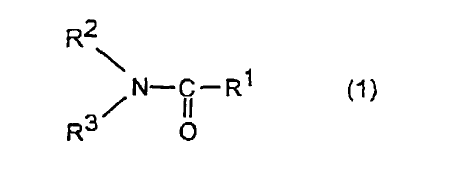 in which R¹, R² and R³, which can be identical or different, represent hydrogen or saturated or unsaturated, aliphatic or cycloaliphatic or cyclic, monovalent hydrocarbonaceous radicals, if functionalized, the functional groups being chosen from esters, ethers, alcohols, amines comprising from 1 to 30 carbon atoms inclusive, it being understood that R¹ can form, either with R² or with R³, a ring comprising from 5 to 18 carbon atoms inclusive and that R² and R³ can together form a ring comprising from 5 to 18 carbon atoms inclusive, with the exception of N,N-disubstituted amide compounds.

2. Composition according to Claim 1, characterized in that the said dibenzoylmethane derivative is chosen from
- 2-methyldibenzoylmethane
- 4-methyldibenzoylmethane
- 4-isopropyldibenzoylmethane
- 4-tert-butyldibenzoylmethane
- 2,4-dimethyldibenzoylmethane
- 2,5-dimethyldibenzoylmethane
- 4,4'-diisopropyldibenzoylmethane
- 4-tert-butyl-4'-methoxydibenzoylmethane
- 2-methyl-5-isopropyl-4'-methoxydibenzoylmethane
- 2-methyl-5-tert-butyl-4'-methoxydibenzoylmethane
- 2,4-dimethyl-4'-methoxydibenzoylmethane
- 2,6-dimethyl-4-tert-butyl-4'-methoxydibenzoylmethane

3. Composition according to Claim 2, characterized in that the said dibenzoylmethane derivative is 4-tert-butyl-4'-methoxydibenzoylmethane or 4-isopropyldibenzoylmethane.

4. Composition according to Claim 3, characterized in that the said dibenzoylmethane derivative is 4-tert-butyl-4'-methoxydibenzoylmethane.

5. Composition according to any one of Claims 1 to 4, characterized in that the amide compound is N-substituted.

6. Composition according to any one of Claims 1 to 5, characterized in that the said monovalent hydrocarbonaceous radicals comprise from 1 to 22 carbon atoms inclusive.

7. Composition according to any one of Claims 1 to 6, characterized in that the R¹ radical is a linear or branched alkyl radical, preferably a C₁-C₁₂ radical, or a phenyl radical optionally substituted by one or more linear or branched C₁-C₁₂ alkyl radicals.

8. Composition according to any one of Claims 1 to 7, characterized in that the R² radical is a linear or branched alkyl radical, preferably a C₁-C₁₂ radical.

9. Composition according to any one of Claims 1 to 7, characterized in that R³ is a linear or branched alkyl radical chosen from those defined for R² or else represents a monovalent radical with an ester functional group corresponding to the following formula (2): 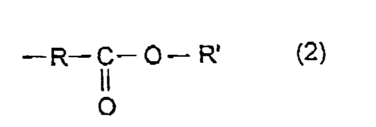 in which R and R', which are identical or different, represent a hydrocarbonaceous radical comprising from 1 to 12 carbon atoms.

10. Composition according to Claim 9, characterized in that the said hydrocarbonaceous radical comprises from 1 to 8 carbon atoms.

11. Composition according to either of Claims 9 and 10, characterized in that the said hydrocarbonaceous radical is of alkyl type.

12. Composition according to any one of Claims 1 to 11, characterized in that the content of dibenzoylmethane derivative(s) is between 0.01% and 10% by weight with respect to the total weight of the composition.

13. Composition according to Claim 12, characterized in that the said content is between 0.1% and 6% by weight with respect to the total weight of the composition.

14. Composition according to any one of Claims 1 to 13, characterized in that the content of amide compound(s) is between 0.01% and 50% by weight with respect to the total weight of the composition.

15. Composition according to Claim 14, characterized in that the said content is between 0.1% and 30% by weight with respect to the total weight of the composition.
